# EUROPEAN PATENT APPLICATION

(11) **EP 2 269 653 A1**
(43) Date of publication of application: **05.01.2011**
(21) Application number: 09724768.8
(22) Date of filing: 25.03.2009
(51) Int. Cl.: A61K 47/32, A61K 9/70, A61K 31/138, A61K 47/10, A61K 47/14, A61P 9/06, A61P 9/10, A61P 9/12, A61P 43/00

(54) **COMPOSITION FOR STABILIZING -BLOCKER, AND TRANSDERMALLY ABSORBABLE PREPARATION COMPRISING THE COMPOSITION**

(30) Priority: 25.03.2008 JP 2008077903
(71) Applicant: Teikoku Seiyaku Co., Ltd., Higashikagawa-shi, Kagawa 769-2695 (JP)
(72) Inventor: ITO, Takeshi, Fukuoka-shi Fukuoka 819-0043 (JP); ISHIGURE, Miho, Higashikagawa-shi Kagawa 769-2695 (JP)
(74) Representative: Ahner, Francis
(86) International application number: PCT/JP2009/055964
(87) International publication number: WO 2009/119672

(57) **Abstract**

The present invention relates to a transdermally absorbable composition which is capable of retaining a β-blocker stably for a long period. More specifically, the present invention relates to a transdermally absorbable composition for stabilizing a β-blocker, which comprises a polymer compound having an amino group, a polyvalent carboxylic acid ester, a fatty acid ester and an acrylic polymer compound.

## Description

### REFERENCE OF RELATED APPLICATIONS

The present application claims the benefit of priority from Japanese Patent Application No. 2008-077903 filed on March 25, 2008, the entire disclosure of which is incorporated herein by reference.

### FIELD OF THE INVENTION

The present invention relates to a composition for stabilizing β-blocker and a transdermally absorbable preparation comprising the composition.

### BACKGROUND OF THE INVENTION

β-blocker is a drug for blocking the β-blocker receptor of sympathetic nerve relating to the exitation of heart and vasoconstriction and mainly used for the treatment or prevention of diseases including essential hypertension, angina pectoris and arrhythmia. Pharmaceutical preparations containing the β-blocker are commercially available primarily in the form of tablets and include, for Example, Maintate^{™} (Mitsubishi Tanabe Parma Corporation, containing bisoprolol fumarate) and Kerlong^{™} (Mitsubishi Tanabe Pharma Corporation, containing betaxolol hydrochloride).

It is important for the therapy and prophylaxis of diseases such as essential hypertension, angina pectoris and arrhythmia to maintain the blood concentration of β-blocker for a long period and to control blood pressure and heart rate. Thus, the development of a transdermally absorbable preparation of a β-blocker has recently attracted a considerable attention in order to administer the β-blocker to a living body for a long period and to control its blood concentration. However, the β-blocker unfortunately tends to be generally decomposed, and thus it often becomes difficult to maintain the β-blocker within the transdermally absorbable preparation for a long period including its shelf life. For instance, it has been described in Japanese Patent Laid-Open Publication No. 2002-308762 that bisoprolol as a β-blocker tends to be hydrolyzed in preparations.

It has also been described in WO2005/072716 that relative humidity is controlled by enclosing a drying agent in a packaging bag in order to improve the storage stability of a transdermally absorbable preparation containing bisoprolol. However, no technique has been described with regard to the improvement of the storage stability of a transdermally absorbable preparation containing a β-blocker.

A transdermally absorbable preparation comprising an adhesive layer containing bisoprolol and polyisobutylene has also been described in WO2007/029781. It has been described that the storage stability of bisoprolol is improved by adding polyisobutylene to the adhesive layer in this transdermally absorbable preparation, However, it seems that β-blocker such as bisoprolol is less soluble in polyisobutylene, which is thus hard to contain a high concentration of a drug required for the administration for a long period,. For instance, in the case of transdermal administration for a long period including 3 days to 1 week, the drug in the transdermally absorbable preparation may be exhausted.
Therefore, it can be said that a composition useful for a transdermally absorbable preparation which comprises a β-blocker in a high dosage suitable for administration for a long period and can be stably retained for a long period is still required.

### SUMMARY OF THE INVENTION

The present inventors have now found that a composition comprising certain components can contain a β-blocker in a high dosage suitable for a long term administration with a transdermally absorbable preparation and can retain the β-blocker stably for a long period. The present invention is based on the findings.
Thus, the object of the present invention is to provide a composition which can comprises a β-blocker in a high dosage suitable for a long term transdermal administration and can retain the β-blocker stably for a long period and a transdermally absorbable preparation containing the composition.

Accordingly, the transdermally absorbable composition for stabilizing β-blocker according to the present invention comprises the β-blocker, a polymeric compound comprising an amino group, a polyvalent carboxylic acid ester, a fatty acid easter, and an acrylic polymer compound.
The transdermally absorbable preparation according to the present invention also comprises at least said composition.

The stabilizing composition according to the present invention has an effect of stabilizing even a β-blocker which tends to be decomposed and can inhibit the decomposition of the β-blocker in the composition remarkably even for a long period of for example one month, so that the composition is convenient for the storage of the transdermally absorbable preparation.
Furthermore, it is possible to retain a high dosage of the β-blocker suitable for the long term administration according to the stabilizing composition of the present invention. Thus, according to the transdermally absorbable preparation having a drug containing layer which comprises the stabilizing composition, the β-blocker can be administered to a living body stably for a long period while controlling the decomposition of the β-blocker, and thus the blood concentration of the β-blocker is advantageously controlled within a certain range for a long period.

### BRIEF DESCRIPTION OF THE DRAWINGS

Figure 1 is a sectional view which illustrates one embodiment of the transdermally absorbable preparation according to the present invention.
Figure 2 is a graph which illustrates the result of the in vitro human skin permeation test with the transdermally absorbable preparation according to the present invention.

### THE DETAILED DESCRIPTION OF THE INVENTION

### DEFINITION

The "alkyl" as used herein means a linear, branched or cyclic alkyl.
Also, the term "alcohol" as used herein means a linear, branched or cyclic saturated or unsaturated alcohol.
Also, the term "higher alcohol" as used herein means an alcohol having six or more carbon atoms.
Also, the term "polyvalent carboxylic acid" as used herein means a bivalent or trivalent carboxylic acid having 6 to 10 carbon atoms.

The composition of the β-blocker according to the present invention is used for stably retaining the β-blocker in the transdermally absorbable preparation as described above and is **characterized in that** the composition comprises the β-blocker, a polymeric compound comprising an amino group, a polyvalent carboxylic acid ester, a fatty acid ester and an acrylic polymer compound. It is an unexpected fact that the above described composition comprising these ingredients is capable of comprising a high dosage of the β-blocker and prominently stabilizing a β-blocker, which is liable to be decomposed.

In the present invention, the β-blocker preferably includes the compounds represented by the following formula (I) or salts thereof.

### [Compound 1]

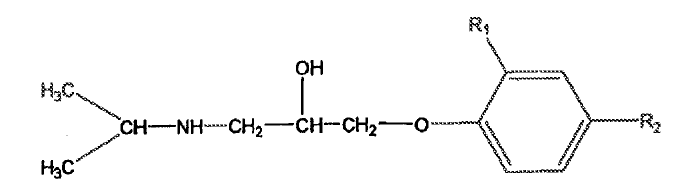

wherein R₁ represents hydrogen or C1-4 alkylcarboxyl group,
R₂ represents a C1-6 alkyloxy C1-3 alkyl group optionally substituted by a C1-5 alkyloxy group,
an aminocarbonyl C1-5 alkyl group,
a C1-6 alkylcarbonyl amino group, or
a C1-3 alkyloxycarbonyl C1-3 alkyl group.

The β-blocker having the structure represented above can be retained stably in the stabilizing composition according to the present invention and includes a β-blocker having a β₁-adrenaline blocking effect. The β-blocker having the β₁-adrenaline blocking effect can be used also in parents suffered from asthma including those whom the blockers of β₂-adrenaline receptor are contraindicant and can be advantageously used also in patients concurrently suffered from hypertension and heart disease.

Further, the C1-4 alkylcarboxyl group represented by R₁ in the compound represented by the formula (I) is preferably a C1-3 alkylcarboxyl group, and more preferably an acetyl group.

Furthermore, the C1-6 alkyloxy C1-3 alkyl group represented by R₂ is preferably a C1-4 alkyloxy C1 - 2 alkyl group, and further preferably a methoxyethyl group, an ethoxymethyl group or a cyclopropylmethoxyethyl group.

Further, one or more hydrogens of the C1-6 alkyloxy C1-3 alkyl group represented by R₂ may be substituted, and such substituents includes a C1-5 alkyloxy group, preferably a C1-3 alkyloxy group, more preferably a methoxy group, an ethoxy group, an n-propoxy group or an isopropoxy group, further preferably an isopropoxy group. In this connection, the C1-5 alkyloxy group as the substituent in the C1-6 alkyloxy C1-3 alkyl group optionally substituted is preferably substituted for one or more hydrogen atoms in the terminal C1-6 alkyl,

Further, the aminocarbonyl C1-5 alkyl group represented by R₂ is preferably an aminocarbonyl C1-3 alkyl group, more preferably an aminocarbonylmethyl group.

Further, the C1-6 alkylcarbonyl amino group represented by R₂ is preferably a C1-3 alkylcarbonylamino group, more preferably a propylcarbonylamino group.

Further, a C1-3 alkyloxycarbonyl C1-3 alkyl group represented by R₂ is preferably a C1-2 alkyloxycarbonyl C1-2 alkyl group, more preferably a methyloxycarbonyl ethyl group.

Moreover, according to the preferred embodiment of the present invention, in the compound represented by the formula (I),
R₁ represents hydrogen or a C1-3 alkylcarboxyl group, and
R₂ represents a C1-4 alkyloxy C1-2 alkyl group optionally substituted by a C1-3 alkyloxy group,
an aminocarbonyl C1-3 alkyl group,
a C1-3 alkylcarbonylamino group, or
a C1 - 2 alkyloxycarbonyl C1-2 alkyl group.

Further according to another embodiment of the present invention, in the compound represented by the formula (I),
R₁ represents hydrogen or an acetyl group, and
R₂ represents a cyclopropylmethoxyethyl group, an isopropoxyethoxymethyl group, a propylcarbonylamino group, an aminocarbonylmethyl group, a methoxycarbonylethyl group or a methoxyethyl group, The compound represented by the formula (I) includes the β-blockers such as betaxolol, bisoprolol, acebutolol, atenolol, esmolol and metoprolol.

Further, in another preferable embodiment of the present invention, in the compound represented by the formula (I), R₁ represents hydrogen, and R₂ represents a C1-4 alkyloxy C1-3 alkyl group optionally substituted by a C1-5 alkyloxy group,
The compound represented by the formula (I) in the stabilizing composition according to the present invention can be prominently stabilized, and its decomposition can be inhibited.

Further, according to another preferred embodiment, in the compound represented by the formula (1), R₂ represents a C1-4 alkyloxy C1 - 2 alkyl group optionally substituted by a C1-3 alkyloxy group.

Furthermore, according to the other further preferred embodiment, in the compound represented by the formula (I), R₂ represents a cyclopropylmethoxyethyl group or an isopropoxyethoxymethyl group. The compound represented by the formula (I) includes β-blockers such as betaxolol or bisoprolol.

Moreover, the salts of the compound represented by the formula (I) preferably include hydrochloride, phthalate, maleate, fumarate, besylate, tosylate, citrate and succinate without limitation thereto.

Furthermore, the stabilizing composition according to the present invention can retain a high dosage of a β-blocker suitable for transdermal administration for a long period, The content of such β-blocker is preferably in the range of 10% by mass or more, more preferably 10 - 40% by mass, further preferably 15 - 30% by mass.

Further, a polymeric compound comprising an amino group in the composition according to the present invention is preferably a C1-6 alkyl (meth)acrylate-C1-6 alkyl (meth)acrylate-di-C1-6 alkylamino C1-6 alkyl (meth)acrylate copolymer, more preferably a C1-3 alkyl (meth)acrylate-C1-3 alkyl (meth)acrylate-di-C1-3 alkylamino C1-3 alkyl (meth)acrylate copolymer, further preferably a methyl (meth)acrylate-butyl (meth)acrylate-dimethylaminoethyl (meth)acrylate copolymer.

The molecular weight (Mw) and the molecular weight distribution of the polymeric compound comprising an amino group can be appropriately determined in consideration of the skin permeation rate, storage period and the like of the β-blocker. Further, the molecular weight (Mw) and the molecular weight distribution of the polymeric compound comprising an amino group can be appropriately adjusted depending on the monomer amount, the molar ratio of the monomer and a polymerisation initiator, the reaction temperature, the solvent and the like, and the molecular weight can be determined for Example by the viscosity method.

Furthermore, the content of the polymeric compound comprising an amino group in the composition according to the present invention is preferably in the range of 5 to 40% by mass, more preferably 8 to 35% by mass, further preferably 12 to 26% by mass.

Further the acrylic polymer compound in the present: invention is preferably a C1-10 alkyl (meth)acrylate-hydroxy C1-6 alkyl (meth)acrylate-C1-6 alkylepoxide (meth)acrylate-vinyl C1-6 carboxylate copolymer, more preferably a C5-8 alkyl (meth)acrylate-hydroxy C1-3 alkyl (meth)acrylate-C1-3 alkylepoxide (meth)acrylate-vinyl C1-3 carboxylate copolymer, further preferably a 2-ethylhexyl acrylate-hydroxyethyl acrylate-glycidyl (meth)acrylate-vinyl acetate copolymer.

Furthermore, the molecular weight (Mw) and the molecular weight distribution of the acrylic polymer compound can be appropriately determined in consideration of the skin permeation rate, the storage period and the like of the β-blocker. In this connection, the molecular weight and the molecular weight distribution of the acrylic polymer compound can be adjusted and determined with the well known technique in the same manner as in the polymeric compound comprising an amino group.

Further, the content of acrylic polymer compound in the composition according to the present invention is preferably in the range of 0.8 to 57% by mass, more preferably 12 to 26% by mass.

Furthermore, the polyvalent carboxylic acid ester in the present invention is preferably a bivalent or trivalent carboxylic acid C1-6 alkyl ester, more preferably a bivalant or trivalent carboxylic acid C1-6 alkyl ester, further preferably a bivalant or trivalent carboxylic acid C1-3 alkyl ester, further preferably a citric acid tri-C1-3 alkyl ester or a sebacic acid di-C1-3 alkyl ester, further preferably triethyl citrate or diethyl sebacate.

Furthermore, the content of the polyvalent carboxylic acid ester in the composition according to the present invention is preferably in the range of 5 to 20% by mass, more preferably 8 to13% by mass.

In addiction, the fatty acid ester in the present invention is a C6-18 fatty acid C1-6 alkyl ester, preferably a C12-16 fatty acid C1-3 alkyl ester More specifically, the fatty acid ester includes hexyl laurate, methyl myristate, isopropyl myristate, isopropyl palmitate or ethy oleate, preferably isopropyl myristate.

It is Preferable that the composition according to the present invention further composes a higher alcohol.
The higher alcohol is preferably a C6-20 alcohol, More specifically, the higher alcohol includes cetyl alcohol, lauryl alcohol, stearyl alcohol, isostearyl alcohol, myristyl alcohol, oleyl alcohol, octyldodecanol or cetostearyl alcohol, preferably oleyl alcohol.

Further, the content of the higher alcohol, in the composition according to the present invention can be set up in the range of about 0 to 10% by mass, preferably 3 to 7% by mass.

Furthermore, according to one embodiment, the combination of the preferred ingredients in the composition described above comprises;
the β-blocker including the compound represented by the formula (I) or a salt thereof, wherein R₁ represents hydrogen or a C1-4 alkylcarbonyl group, R₂ represents a C1-6 alkyloxy C1-3 alkyl group optionally substituted by a C1-5 alkyloxy group, an aminocarbonyl C1-5 alkyl group, a C1-6 alkylcarbonyl amino group, or a C1-3 alkyloxy carboxyl C1-3 alkyl group;
the polymeric compound comprising an amino group including a C1-6 alkyl (meth)acrylate-C1-6 alkyl (meth)acrylate-di-C1-6 alkylamino C1-6 alkyl (meth)acrylate copolymer,
the polyvalent carboxylic acid ester including a bivalent or trivalent carboxylic acid C1-6 alkyl ester,
the fatty acid ester including a C4-18 fatty acid C1-6 alkyl ester, and
the acrylic polymer compound including a C1-10 alkyl (meth)acrylate-hydroxy C1-6 alkyl (meth)acrylate-C1-6 a!kylepoxide (meth)acrylate-vinyl C1-6 carboxylate copolymer.

Furthermore, according to the more preferred embodiment of the present invention, in the composition described above
the β-blocker represents said compound represented by the formula (I) or a salt thereof,
the polymeric compound comprising an amino group represents a C1-6 alkyl (meth)acrylate-C1-6 alkyl (meth)acrylate-di-C1-6 alkylamino C1-6 alkyl (meth)acrylate copolymer,
the polyvalent carboxylic acid ester represents a bivalent or trivalent carboxylic acid C1-6 alkyl ester,
the fatty acid ester represents a C12-16 fatty acid C1-3 alkyl ester, and
the acrylic polymer compound represents a C1-10 alkyl (nmeth)acrylate-hydroxy C1-6 alkyl (meth)acrylate-C1-6 alkylepoxide (meth)acrylate-vinyl C1-6 carboxylate copolymer.

Furthermore, according to the further preferred embodiment of the present invention, in the composition described above
the β-blocker represents said compound represented by the formula (I) or a salt thereof,
the polymeric compound comprising an amino group represents a C1-6 alkyl (meth)acrylate-C1-6 alkyl (meth)acrylate-di-C1-6 alkylmino C1-6 alkyl (meth)acrylate copolymer,
the polyvalent carboxylic acid ester represents a citric acid di-C1-3 alkyl ester or a sebacic acid tri- C1-3 alkyl ester,
the fatty acid ester represents a C4-18 fatty acid C1-6 alkyl ester, and
the acrylic polymer compound represents a C1-10 alkyl (meth)acrylate-hydroxy C1-6 alkyl (meth)acrylate-C1-6
alkylepoxide (meth)acrylate-vinyl C1-6 carboxylate copolymer.

Furthermore, according to the further preferred embodiment of the present invention, in the composition described above
the β-blocker represents said compound represented by the formula (I) or a salt thereof,
the polymeric compound comprising an amino group represents a C1-6 alkyl (meth)acrylate-C1-6 alkyl (meth)acrylate-di-C1-6 alkylamino C1-6 alkyl (meth)acrylate copolymer,
the polyvalent carboxylic acid ester represents a citric acid di-C1-3 alkyl ester or a sebacic acid tri- C1-3 alkyl ester,
the fatty acid ester represents a C12-16 fatty acid C1-3 alkyl ester, and
the acrylic polymer compound represents a C5-8 alkyl (meth)acrylate-hydroxy C1-3 alkyl (meth)acrylate-C1-3 alkylepoxide (meth)acrylate-vinyl C1-3 carboxylate copolymer,

In addition, according to the further preferred embodiment of the present invention, in the composition described above
the β-blocker represents the compound represented by the formula (I) or a salt thereof,
the polymeric compound comprising an amino group represents a methyl (meth)acrylate-butyl (meth)acrylate-dimethylaminoethyl (meth)acrylate copolymer,
the polyvalent carboxylic acid ester represents triethyl citrate,
the fatty acid ester represents isopropyl myristate, and
the acrylic polymer compound represents 2-ethylhexyl acrylate- hydroxyethyl acrylate-glycidyl (meth)acrylate-vinyl acetate copolymer.

Furthermore, according to the further preferred embodiment, in the compound represented by the formula (I) in the composition described above, R₁ represents hydrogen or a C1-3 alkylcarboxyl group, and R₂ represents a C1-4 alkyloxy C1 - 2 alkyl group optionally substituted by a C1-3 alkyloxy group, an aminocarbonyl C1-3 alkyl group, a C1-3 alkylcarbonyl amino group, or C1-2 alkyloxy carbonyl C1-2 alkyl group.

Furthermore, according to the further preferred embodiment, in the composition describe above, R₁ represents hydrogen, and R₂ represents a C1-4 alkyloxy C1-3 alkyl group optionally substituted by a C1-5 alkyloxy group.

Furthermore, according to the further preferred embodiment, in the composition described above, R₁ represents hydrogen, and R₂ represents a C1-4 alkyloxy C1-2 alkyl group optionally substituted by a C1-3 alkyloxy group.

In addition, according to the further preferred embodiment, in the composition described above, R₁ represents hydrogen or an acetyl group, and R₂ represents a cyclopropylmethoxyethyl group, an isopropoxyethoxymethyl group, a propylcarbonyl amino group, an aminocarbonylmethyl group, a methoxycarbonylethyl group or a methoxyethyl group.

In addition, according to the further preferred embodiment, in the composition, R₁ represents hydrogen, and R₂ represents a cyclopropylmethoxyethyl group or an isopropoxyethoxymethyl group. In this embodiment, the β-blocker corresponds to betaxolol or bisoprolol and are remarkably stabilized and inhibited from decomposition, so that these β-blockers can be stored stably for a long period.
In this connection, the combination of the preferred ingredients described above may further contain the aforementioned higher alcohol, preferably a C6-20 alcohol, more preferably cetyl alcohol, lauryl alcohol, stearyl alcohol, isostearyl alcohol, myristyl alcohol, oleyl alcohol, octyl dodecanol or cetostearyl alcohol, further preferably oleyl alcohol.

### Transdermally absorbable preparation

The composition according to the present invention can contain a high dosage of a β-blocker as described above and stably retain the β-blocker for a long period. Thus, the use of such composition as the drug containing layer of a transdermally absorbable preparation is convenient for efficiently administering the β-blocker to the skin for a long period. Thus, according to one embodiment, the transdermally absorbable preparation comprises at least one drug containing layer comprising the composition according to the present invention. In addition, such drug containing layer may be used directly as a transdermally absorbable preparation, but it is desirable to dispose on the skin-contacting side a drug permeable polymer membrane which ensures the controlled release of the β-blocker into the skin, taking the release control of the β-blocker into account. Furthermore, it is preferable to dispose the drug permeable polymer membrane so that it is in direct contact with the skin in the transdermally absorbable preparations. Such a disposition of the drug permeable polymer membrane is advantageous to retaining the skin permeation rate of a drug for a long period and efficiently administering the drug to the skin. Thus, according to the preferred embodiment, the transdermally absorbable preparation comprises a laminate comprising, in the order from the skin side, at least an outer membrane and the drug containing layer, and a fixing means for fixing the laminate on the skin, wherein the drug containing layer comprises the composition according to the present invention and the outer membrane is a drug permeable polymer membrane which ensures controlled release of the β-blocker into the skin and provided on the skin- contacting surface of the laminated.

The preferred embodiment of the transdermally absorbable preparation according to the present invention is illustrated with reference to the scheme in the following,

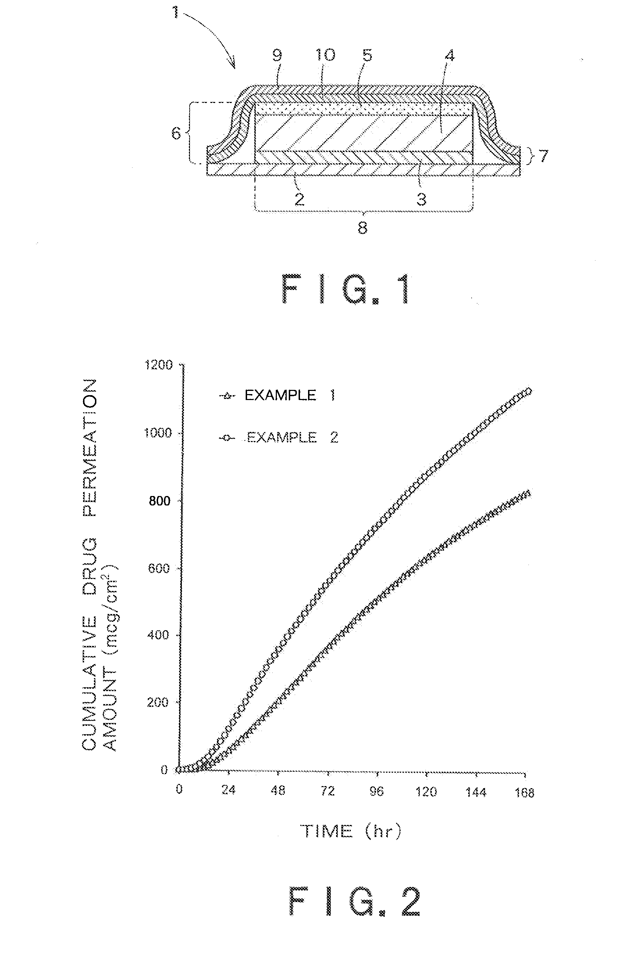

Figure 1 is a sectional view illustrating an embodiment of the transdermally absorbable preparation according to the present invention,
As shown in Figure 1, the transdermally absorbable preparation 1 is provided with a laminate which comprises an outer membrane 3, a drug containing layer 4 and a support layer 5, and a fixing means 7 for fixing the laminate 6 on the skin 2. In addition, the outer membrane 3 comprises a drug permeable polymer membrane ensures controlled release of the β-blocker into the skin 2 and is provided on the skin-contacting surface 8 of the laminate 6.

In addition, the fixing means 7 comprises the cover layer 9 covering the laminate 6 and the adhesive layer 10 for adhering the cover layer 9 to the skin. The cover layer 9 covers the part except the skin-contacting surface 8 of the laminate 6. In addition, the adhesive layer 10 is disposed on the skin side of the cover layer 9. Furthermore, the adhesive layer 10 is disposed in the peripheral and terminal regions in the skin-contacting surface 8 of the laminate 6 and fixes the transdermally absorbable preparation 1 on the skin 2. Such arrangement of the adhesive layer is advantageous to avoiding the transfer of the components including the drug resulting in the change of the physical properties of the adhesive layer with the passage of time and retaining the stable adhesion of the transdermally absorbable preparations to the skin.
In this connection, it is also possible to further coat a part of the skin side of the drug permeable membrane with an adhesive as far as the drug permeable membrane can be directly contacted with the skin in order to assist the adhesion of the transdermally absorbable preparation and the skin. The present invention also involves such embodiment.

Furthermore, the drug permeable polymer membrane composing the outer membrane in the transdermally absorbable preparation according to the present invention is preferably a microporous membrane having drug permeable pores, but the membrane is not limited thereto as far as it ensures controlled release of the drug to the skin. The microporous membrane is advantageous to adjusting the drug release rate and controlling the blood concentration of the drug within the safe and effective range. The pore size and the pore density of the microporous membrane can be determined appropriately in consideration of for example the desired skin permeation rate of the β-blocker. Also, the area of the skin side of the drug permeable membrane can be appropriately determined in consideration of for example the desired skin permeation rate, application site and the like.

Furthermore, the preferred exampes of the constituent materials of the drug permeable polymer membrane include EVA (ethylene-vinyl acetate copolymer), polyethylene, polypropylene, polyacrylonitrile, polymethyl methacrylate, and a combination thereof, preferably polypropylene or EVA.

In addition, the adhesive layer is not particularly limited to as far as it is a biocompatible material being capable of bonding the skin and the transdermally absorbable preparation and preferably includes polyacrylate, polydimethylsiloxane, polyisobutylene or a combination thereof. Furthermore, to the constituent materials of the adhesive layer may be added appropriately for example a well known tackifier, and the like. The aforementioned materials can be also used as an auxiliary adhesive added to the surface of the drug permeable membrane.

Also, the contact area of the adhesive layer to the the skin can be appropriately determined in consideration of the area of the drug permeable membrane, the dosage period, the application site, and the like.

In addition, the support layer may be stretchable or non-stretchable. The specific material constituting the support layer includes, but is not limited to as far as it can separate the drug containing layer from the other materials, for instance, woven fabric, unwoven fabric, PET (polyethylene terephthalate), polyurethane, polyester, polyethylene or a composite material thereof.
Also, for the cover, a similar material to that of the support layer may be used.

### Preparation process

The preferred process for producing the transdermally absorbable preparation according to the present invention is described below.
The components of the stabilizing composition according to the present invention are appropriately mixed in a solvent to prepare a mixture containing the stabilizing composition in the solvent. Next, the mixture is used as a plaster solution and coated on a liner. Next, the plaster solution is dried at a temperature of about 60 to 120°C to give a drug containing layer, on which a support layer is laminated. Next, the liner is removed from the drug containing layer, and the drug permeable membrane is laminated on the side of the drug containing layer on which the liner had been disposed to give a laminate. Next, a cover having an adhesive layer disposed on one side is provided. Next, the adhesive layer side of the cover and the support layer side of the laminate are sticked together to give a transdermally absorbable preparation. In this connection, the position and the size of the adhesive layer are preliminarily configured so that the adhesive layer covers the peripheral or terminal regions of one skin side of the drug permeable membrane.

In the preparation process described above, the solvents used in preparing the drug containing layer and the adhesive layer include, for example, ethyl acetate, butyl acetate, toluene, n-hexane, n-heptane, tetrahydrofuran, dimethylformamide, methanol or ethanol.

### Use

The transdermally absorbable preparation according to the present invention can make a drug administered stably and efficiently to a living body. Thugs, the application period of the transdermally absorbable preparation can be established to a long period even on a single administration, preferably for 3 to 7 days, more preferably about 7 days. Also, the β-blocker in the transdermally absorbable preparation is prominently stabilized, and the transdermally absorbable preparation can be stably stored for at least a month even in the absence of a desiccant.

Further, object disorders can be appropriately selected depending on the kinds or characters of drugs, and the administration schedule of a drug is appropriately determined depending on the kind of the drug, the condition of a patient, the administration period, the size of the drug, and the like by a person skilled in the art.

Furthermore, the living bodies to which the transdermally absorbable preparation according to the present invention is applied include, for example, rabbit, dog or human, preferably human.

### Example

The present invention is now described specifically with reference to examples, but it is not limited to these examples.

### Example 1

### Formulation

**[Table 1]**

| Component % by mass | |
|---|---|
| Betaxolol hydrochloride (β-blocker) | 20 |
| Aminoalkyl methacrylate copolymer | 17.1 |
| Triethyl citrate | 10 |
| Isopropyl myristate | 10 |
| Oley alcohol | 5 |
| Duro-Tak^{®}387-2516 | 37.9 |

| | |
|---|---|
| Outer membrane: Ethylene-vinyl acetate copolymer membrane (CoTran^{™} 9702, 3M) | |

Betaxolol hydrochloride (LUSOCHIMICA S.P.A), aminoalkyl methacrylate copolymer E (Degussa), triethyl citrate (Wako Pure Chemical) Industries, Ltd.), isopropyl myristate (Nikko Chemicals) and oleyl alcohol (Kokyu Alcohol Kogyo Co., Ltd.) were provided in the proportion described in the formulation and mixed by agitation in an appropriate amount of ethyl acetate. To the mixture thus obtained was added Duro-Tak^{™} 387-2516 (National Starch & Chemical) in a proportion described in the formulation to give a plaster solution.
The plaster solution was coated on a polyethylene terephthalate liner and dried at 70°C for 15 minutes to give a drug containing layer. The drug containing layer after drying was adjusted to a weight of 100 g/m².

Next, a support layer (Scotchpak^{™} 9732, 3M) was laminated on the side opposite to the liner of the drug containing layer. The liner was then removed from the drug containing layer and the drug containing layer was sticked to an ethylene-vinyl acetate copolymer membrane (outer membrane) to give a transdermally absorbable preparation.

### Example 2

### Formulation

**[Table 2]**

| Component | % by mass |
|---|---|
| Bisoprolol fumarate(β-blocker) | 30 |
| Aminoalkyl methacrylate copolymerE | 24.1 |
| Triethyl citrate | 10 |
| Isopropyl miristate | 10 |
| Duro-Tak^{™} 387-2516 | 25.9 |

| | |
|---|---|
| Outer membrane: Microporous polypropylene membrane (Celgard^{™} 2400, Celgard) | |

Bisoprolol fumarate (Parmachem Asia), aminoalkyl methacrylate copolymer E (Degussa), triethyl citrate (Wako Pure Chemical Industries, Ltd.), isopropyl myristate (Nikko Chemicals Co., Ltd.) and oleyl alcohol (Kokyu Alcohol Kogyo Co., Ltd.) were provided in the proportion described in the formulation and mixed by agitation in an appropriate amount of ethyl acetate. To the mixture thus obtained was added Duro-Tak^{™} 387-2516 (National Starch & Chemical) in a proportion described in the formulation to give a plaster solution.
The plaster solution was coated on a polyethylene terephthalate liner and dried at 70°C for 15 minutes to give a drug containing layer. The drug containing layer after drying was adjusted to a weight of 100 g/m².

Next, a support layer (Scotchpak^{™} 9732, 3M) was laminated on the reverse side to the liner of the drug containing layer, The liner was then removed from the drug containing layer and the drug containing layer was sticked to a microporous polypropylene membrane (outer membrane) to give a laminate (10 cm²). Next, a foam tape (9773, 3M, 21.3 cm²) was provided as a cover, and the adhesive layer side of the foam tape and the support layer side of the laminate were sticked together to give a transdermally absorbable preparation.

### Example 3

### Formulation

**[Table 3]**

| Component | % by mass |
|---|---|
| Bisoprolol fumarate (β-blocker) | 25 |
| Aminoalkyl methacrylate copolymer E | 20 |
| Triethyl citrate | 10 |
| Isopropyl myristate | 10 |
| Oleyl alcohol | 5 |
| Duro-Tak^{™} 387-2516 | 30 |

| | |
|---|---|
| Outer membrane: Microporous polypropylene membrane Outer membrane: Microporous polypropylene membrane (Celgard^{™} 2400, Celgard) | |

Bisoprolol fumarate, aminoalkyl methacrylate copolymerE, triethyl citrate, isopropyl myristate and oleyl alcohol were provided in the proportion described in the formulation and mixed by agitation in an appropriate amount of ethyl acetate. To the mixture thus obtained was added Duro-Tak^{™} 387-2516 (National Starch & Chemical) in a proportion described in the Formulation to give a plaster solution.
The plaster solution was coated on a polyethylene terephthalate liner and dried at 70°C for 15 minutes to give a drug containing layer. The drug containing layer after drying was adjusted to a weight of 100 g/m².

Next, a support layer (Scotchpak^{™} 9732, 3M) was laminated on the reverse side to the liner of the drug containing layer. The liner was then removed from the drug containing layer and the the drug containing layer was sticked to a microporous polypropylene membrane (outer membrane) to gave a laminate (10 cm²). Next, a foam tape (9773, 3M, 21.3 cm²) was provided as a cover, and the adhesive layer side of the foam tape and the support layer side of the laminate were sticked together to give a transdermally absorbable preparation.

### Referential Example 1

### Formulation

**[Table 4]**

| Component | % by mass |
|---|---|
| Betaxolol hydrochloride (β-blocker) | 5 |
| Diisopropanolamine | 2 |
| Liquid paraffin | 23.3 |
| Oppanol B100 | 1.7 |
| Oppanol B10 | 5.2 |
| Styrene-isoprene-styrene block copolymer | 16.3 |
| Alicyclic saturated hydrocarbon resin | 46.5 |

Betaxolol hydrochloride, diisopropanolamine and liquid paraffin were mixed by agitation in the proportion described in the formulation to give a mixed solution, Next, a solution having Oppanol B100 (BASF), Oppanol B10 (BASF), a styrene-isoprene-styrene block copolymer (QUINTAC 3570C, Zeon Corporation) and an alicyclic saturated hydrocarbon resin (ARKON P-100, Arakawa Chemical Industries, Ltd.) dissolved in toluene was provided and added to the mixed solution described above, and the mixture was agitated to give a coating solution which contains the respective ingredients in a proportion described in the formulation. The coating solution was coated on a polyester liner and dried at 80°C for 15 minutes to give a drug containing layer. The drug containing layer after drying was adjusted to a thickness of 100 µm. Next, a polyester film (Scotchpak^{™} 9732, 3M) as a support layer was laminated on the reverse side to the liner of the drug containing layer to give a matrix type transdermally absorbable preparation.

### Referential Example 2

### Formulation

**[Table 5]**

| Component | % by mass |
|---|---|
| Bisoprolol fumarate (β-blocker) | 10 |
| Triethanolamine | 7.8 |
| Liquid paraffin | 5 |
| Isopropyl myristate | 5 |
| Styrene-isoprene-styrene block copolymer | 24.1 |
| Alicyclic saturated hydrocarbon resin | 48.2 |

Bisoprolol fumarate, triethanolamine, liquid paraffin and isopropyl myristate were mixed by agitation in the proportion described in the formulation to give a mixed solution. Next, a solution having a styrene-isoprene-styrene block copolymer and an alicyclic saturated hydrocarbon resin dissolved in toluene was provided and added to the mixed solution described above, and the mixture was agitated to give a coating solution which contains the respective ingredients in a proportion described in the formulation. The coating solution was coated on a polyester liner and dried at 80°C for 15 minutes to give a drug containing layer. The drug containing layer after drying was adjusted to a thickness of 100 µm. Next, a polyester film (Scotchpak^{™} 9732, 3M) as a support layer was laminated on the reverse side to the liner of the drug containing layer to give a matrix type transdermally absorbable preparation. In addition, the liner was removed from the ransdermally absorbable preparation when the preparation is used,

### Test Example 1

### In vitro human skin permeation test

A part of a laminate obtained by bonding the drug containing layer and the outer membrane in Examples 1 and 2 was placed on a corneal layer of human epidermis so that the outer membrane is arranged on the skin- contacting surface (application area: 4.5cm²). The sample thus obtained was set up on a flow-through-cell through which warm water was circulated so that the skin surface is maintained at a temperature of about 32°C. Phosphate buffer physiological saline (pH 7.4) was used as a receiver solution, which was collected every 2 hours in an amount of 5ml/hr for 24 hours. The flow rate of the solution thus collected was measured, and the cumulative drug permeation amount of a β-blocker was measured by HPLC. The skin permeation rate per hour was calculated from the results thus obtained.

The result is shown in Figure 2. It has been confirmed that both of the transdermally absorbable preparation in Examples 1 and 2 release the drug in an approximately constant skin permeation rate for 1 week,
Further, the skin permeation rates (mcg/cm²/hr) in the stationary state of the transdermally absorbable preparations in Examples 1 and 2 are shown in Table 1.

### [Table 6]

**Table 1**

| Preparation | Skin permeation rate (mcg/cm²/hr) |
|---|---|
| Example 1 | 2.75 |
| Example 2 | 10.50 |

### Test Example 2

### Drug storage stability test

The transdermally absorbable preparations of Examples -1 to 3 were respectively enclosed in aluminum packaging bags for storing at 40°C for one month. The transdermally absorbable preparations of Referencial Examples 1 and 2 were respectively enclosed in aluminum packaging bags for storing at 40°C for one month in the same manner as those of Examples -1 to 3.

Then, the contents of the drug in the respective transdermally absorbable preparations were measured by HPLC for calculating the ratio of the drug content at the end of the test to the drug content at the initiation of the test.
The results are shown in Table 2. About 5% of the decrease in the content of the drug was observed in Referential Examples 1 and 2 at 1 month after initiating the test. On the other hand, the content of the drug was in the range of 101.0 - 99.7 in Example -1 to 3, and no decrease in the content of the drug was observed.
In this connection, the standard error in this test was about 2% (n=5).

### [Table 7]

**Table 2**

| | Content of drug (%) |
|---|---|
| Example 1 101.0 | |
| Example | 2 99.7 |
| Example 3 101.0 | |
| Example 4 94.6 | |
| Example 5 | 94.7 |

### Test Example 3

### Drug storage stability test

Storage stability at 40°C for 6 months was confirmed in the same technique as in Test Example 2 with regard to the transdermally absorbable preparations of Examples 1 and 3.

The results are shown in Table 3. The contents of the drugs in Examples 1 and 3 were 100.1% and 98.4%, respectively, which were higher than those in Referential Example 1 and 2 at 1 month after initiating the test in Test Example 2.
In this connection, the standard error in this test was about 2% (n=5).

### [Table 8]

**Table 3**

| | Content of drug (%) |
|---|---|
| | Example 1 100.0 |
| Example 3 98.4 | |

## Claims

1. A composition for stabilizing a β-blocker, which comprises the β-blocker, a polymeric compound comprising an amino group, a polyvalent carboxylic acid ester, a fatty acid ester and an acrylic polymer compound.

2. A composition according to Claim 1 for the transdermally absorbable preparation.

3. A composition according to Claim 1, wherein said β-blocker is the compound represented by the formula (I) or a salt thereof: 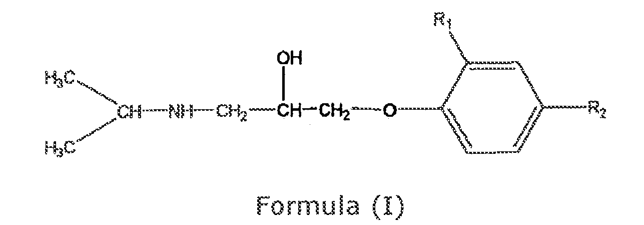 wherein R₁ represents hydrogen or a C1-4 alkylcarboxyl group,
R₂ represents a C1-6 alkyloxy C1-3 alkyl group optionally substituted by a C1-5 alkyloxy group,
an aminocarbonyl C1-5 alkyl group,
a C1-6 alkylcarbonyl amino group, or
a C1-3 alkyloxy carbonyl C1-3 alkyl group.

4. A composition according to Claim 3, wherein R₁ represents hydrogen, and R₂ represents a C1-4 alkyloxy C1-3 alkyl group optionally substituted by a C1-5 alkyloxy group.

5. A composition according to Claim 4, wherein R₁ represents hydrogen, and
R₂ represents a cyclopropylmethoxyethyl group or an isopropoxyethoxymethyl group.

6. A composition according to Clam 1, wherein the content of said β-blocker is in the range of 10% by mass or more.

7. A composition according to Claim 1, wherein the content of said β-blockeris in the range of 15 - 30% by mass or more.

8. A composition according to Claim 1, wherein said polymeric compound comprising an amino group is a C1-6 alkyl (meth)acrylate - C1-6 alkyl (meth)acrylate - di-C1-6 alkylamino C1-6 alkyl (meth)acrylate copolymer.

9. A composition according to Claim 1, wherein said polymeric compound comprising an amino group is a C1-3 alkyl (meth)acrylate - C1-3 alkyl (meth)acrylate - di-C1-3 alkylamino C1-3 alkyl (meth)acrylate copolymer.

10. A composition according to Claim 1, wherein said polyvalent carboxylic acid ester is a bivalent or trivalent carboxylic acid C1-6 alkyl ester.

11. A composition according to Claim 10, wherein said polyvalent carboxylic acid ester is a citric acid di-C1-3 alkyl ester or sebacic acid tri-C1-3 alkyl ester,

12. A composition according to Claim 1, wherein said fatty acid ester is a C4 - 18 fatty acid C1-6 alkyl ester.

13. A composition according to Claim 12, wherein said fatty acid ester is a C12 - 16 fatty acid C1-3 alkyl ester.

14. A composition according to Claim 1, wherein said acrylic polymer compound is a C1 - 10 alkyl (meth)acrylate-hydroxy C1-6 alkyl (meth)acrylate-C1-6 alkylepoxide (meth)acrylate-vinyl C1-6 carboxylate copolymer.

15. A composition according to Claim 14, wherein said acrylic polymer compound is a C5-8 alkyl (meth)acrylate-hydroxy C1-3 alkyl (meth)acrylate-C1-3 alkyl epoxide (meth)acrylate-vinyl C1-3 carboxylate copolymer.

16. A composition according to Claim 1, further comprising a higher alcohol.

17. A composition according to Claim 16, wherein said higher alcohol a C6-20 alcohol.

18. A composition according to Claim 16, wherein said higher alcohol is an oleyl alcohol.

19. A composition according to Claim 1, wherein said β-blocker is bisoprolol, betaxolol or a salt thereof,
said polymeric compound comprising an amino group is a methyl (meth)acrylate-butyl (meth)acrylate-dimethylaminoethyl (meth)acrylate copolymer,
said polyvalent carboxylic acid ester is diethyl citrate, said fatty acid ester is isopropyl myristate, and
said acrylic polymer compound is a 2-ethylhexyl acrylate-hydroxyethyl acrylate-glycidyl (meth)acrylate-vinyl acetate copolymer.

20. A transdermally absorbable preparation comprising,
a laminate comprising, in the order from the side-contacting side, an outer membrane and a drug containing layer,
a fixing means for fixing said laminate on the skin,
wherein said drug containing layer comprises a composition according to Claim 1, and wherein said outer membrane is a drug permeable polymer membrane which ensures controlled release of the β-blocker into the skin and is provided on the skin-contacting surface of the laminate.

21. A transdermally absorbable preparation according to Claim 20, wherein said outer membrane is a microporous membrane having β -blocker permeable pores.

22. A transdermally absorbable preparation according to Claim 20, wherein said outer membrane comprises a polypropylene or ethylene-vinyl acetate copolymer.
